(19) Europäisches Patentamt
European Patent Office
Office européen des brevets

(11) Publication number: **0 025 288**
**B1**

(12) # EUROPEAN PATENT SPECIFICATION

(45) Date of publication of patent specification: **06.04.83**

(21) Application number: **80302838.0**

(22) Date of filing: **18.08.80**

(51) Int. Cl.³: **C 07 D 309/10,**
C 07 D 407/06,
C 07 D 409/14,
A 61 K 31/335,
A 61 K 31/38

(54) **Derivatives of monic acid, processes for their preparation, compositions containing them and their preparation.**

(30) Priority: **31.08.79 GB 7930293**

(43) Date of publication of application:
**18.03.81 Bulletin 81/11**

(45) Publication of the grant of the patent:
**06.04.83 Bulletin 83/14**

(84) Designated Contracting States:
**BE CH DE FR GB IT LI NL SE**

(56) References cited:
**GB - A - 2 009 154**

(73) Proprietor: **BEECHAM GROUP PLC**
**Beecham House Great West Road**
**Brentford Middlesex (GB)**

(72) Inventor: **Rogers, Norman Harold**
**6 The Marts**
**Rudgwick Sussex (GB)**
Inventor: **Coulton, Steven**
**6 Coatham Place**
**Cranleigh Surrey (GB)**

(74) Representative: **Cresswell, Thomas Anthony et al,**
**European Patent Attorney Beecham**
**Pharmaceuticals Great Burgh Yew Tree Bottom Road**
**Epsom Surrey KT18 5XQ (GB)**

**The file contains technical information submitted after the application was filed and not included in this specification**

**EP 0 025 288 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Antibacterial compounds, processes for their preparation and compositions containing them

This invention relates to antibacterial compounds and in particular to a class of ketones which have antibacterial activity against certain Gram-positive and Gram-negative organisms, and also possess anti-myco-plasmal activity. The compounds are therefore of value in the treatment of human and veterinary infections.

The compounds of formula (I) and salts and esters thereof:

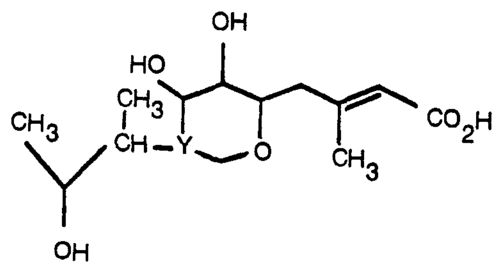

(I)

wherein Y represents

$—CH=CH—CH_2—CH—$,

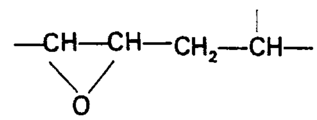

and

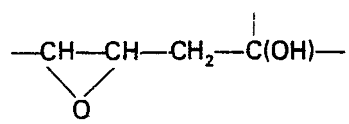

are disclosed in West German Offenlegungsschriften No. 2726619, 2726618 and 2848687 and European Patent Application No. 79300371.6. Compounds of formula (I) having the tri-substituted double bond in the E-configuration are referred to as monic acid C, monic acid A and monic acid B respectively.

The present invention provides a ketonic ester of formula (II):

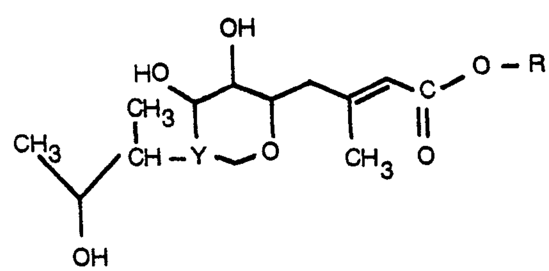

(II)

wherein Y is as defined with respect to formula (I); and R represents a $C_{3-20}$ alkyl, $C_{3-8}$ cycloalkyl, $C_{4-20}$ alkenyl, aralkyl, cycloalkylalkyl, heterocyclyl or heteroclylalkyl group, which group is substituted with a ketonic oxo group. Preferably the compounds of formula (II) are derivatives of monic acid A, i.e. Y represents

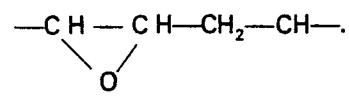

Suitably the group R represents $C_{3-10}$ alkyl group, a heterocyclylalkyl group or a $C_{5-8}$ cycloalkyl group, each of which is substituted with a ketonic oxo group.

The compound (II) of this invention incorporates a tri-substituted double bond and may therefore exist in both the E (natural) and Z (or iso) geometrical forms. It is to be understood that both geometrical isomers of the compound of formula (II) are included within the scope of this invention, as well as mixtures of the two isomers. However, because in general the E-isomer of a particular derivative of compound (II) has the greater activity, it is preferable to employ that isomer.

In addition, the 10, 11 double bond in the C-series of compounds is naturally E in configuration.

One sub-class of compounds of this invention comprises compounds of formula (III):

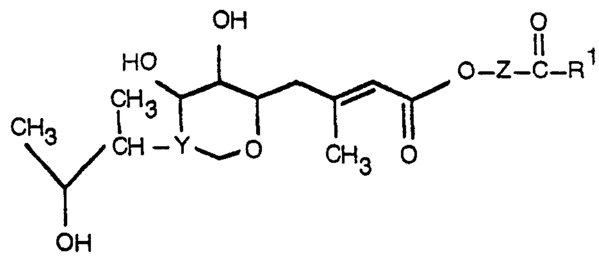

(III)

wherein Y is as defined with respect to formula (I); Z represents straight or branched chain $C_{1-12}$ alkylene; and $R^1$ represents a $C_{1-10}$ alkyl, $C_{3-8}$ cycloalkyl, $C_{2-10}$ alkenyl, aryl, aralkyl, cycloalkylalkyl or heterocyclyl group. Suitably $R^1$ represents $C_{1-8}$ alkyl or heterocyclyl.

Preferably Z represents straight chain $C_{1-12}$ alkylene, in particular Z represents methylene, propylene, pentylene or octylene and $R^1$ represents methyl, 2-thienyl, phenyl or substituted phenyl.

Particular compounds within formula (III) include acetylmethyl monate A, 3-acetylpropyl monate A, 8-acetyloctyl monate A, 4(1-thienyl-2-yl-butan-1-onyl)monate A, 4-phenylbutan-4-oxyl monate A, 5-acetylpentyl monate A and 4-(4-acetamidophenyl)-4-oxobutyl monate A.

Compounds of this invention have antibacterial and antimycoplasmal activity, and are therefore of value in the treatment of bacterial and mycoplasma-induced human and veterinary diseases.

The infections against which compounds of this invention are particularly useful include venereal disease. They are also effective in the treatment of respiratory infections such as bacterial bronchitis; and bacterial meningitis, non-specific urethritis and pseumonia. In animals it may be employed for the treatment of mastitis in cattle, for swine dysentery, and for mycoplasma infections in animals such as turkeys, chickens, pigs and cattle.

Some of the human and veterinary diseases either caused by mycoplasma species or in which they play a prominent role, and against which compounds of this invention are effective, are as follows:

*Avian*

*M gallisepticum* — Chronic respiratory diseases (air-sacculitis of chickens and turkeys

*M synoviae* — Airsacculitis and infections synovitis

*Bovine*

*M bovis* — Mastitis, respiratory disease and arthritis of cattle

*M dispar* — Calf pneumonia

*Porcine*

*M suipneumoniae* — Enzootic pneumonia of pigs

*M hyorhinis*
*M hyosynoviae* } — arthritis in pigs

*Murine*

*M pulmonis* — pneumonia of rats and mice

*M arthritidis* — arthritis in rats and mice

*Human*

*M pneumoniae* — Primary atypical pneumonia

Compounds of the present invention are particularly useful in the treatment of enzootic pneumonia in animals such as pigs, cattle and sheep, because they also have activity against the bacteria Bordetella bronchispetica, Pasteurella multocida and Haemophilus spp, which often causes respiratory complications in cases of this disease.

This invention also provides a pharmaceutical or veterinary composition which comprises a compound of formula (II) togeter with a pharmaceutically or veterinary acceptable carrier or excipient.

The compositions may be formulated for administration by any route, and would depend on the disease being treated. The compositions may be in the form of tablets, capsules, powders, granules, lozenges, liquid or gel preparations, such as oral, topical or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinyl-pyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquids preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non aqueous vehicles (which may include edible oils), for example almond oil, fractionated coconut oil, oily esters such as glycerine, propylene glycol, or ethyl alcohol; preservatives, for example methyl or propyl *p*-hydroxybenzoate or sorbic acid, and if desired conventional flavouring or colouring agents.

For topical application to the skin compounds of this invention may be made up into a cream, lotion or ointment. Cream or ointment formulations that may be used for compounds of formula (II) are conventional formulations well known in the art, for example, as described in standard text books of pharmaceutics and cosmetics, such as Harry's Cosmeticology published by Leonard Hill Books, and the British Pharmacopoeia.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can either be suspended or dissolved in the vehicle. In preparing solutions the compound can be dissolved in water for injection and filter sterilized before filling into a suitable vial or ampoule and sealing. Advantageously adjuvants such as a local anaesthetic, preservative and buffering agents can be dissolved in the vehicle. To enhance the stability the composition can be frozen after filling into the vial and water removed under vacuum. The dry lyophilized powder is then sealed in the vial. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compounds.

Veterinary compositions for intramammary treatment of mammary disorders in animals, especially bovine mastitis, will generally contain a suspension of a compound of formula (II) in an oily vehicle.

The composition may contain from 0.1% to 99% by weight, preferably from 10—60% by weight, of the active material, depending on the method of administration. Where the compositions comprise dosage units, each unit will preferably contain from 50—500 mg, of the active ingredient. The dosage as employed for adult human treatment will preferably range from 100 mg to 3 g, per day, for instance 250 mg to 3 g, per day, depending on the route and frequency of administration.

Alternatively a compound of formula (II) may be administered as part of the total dietary intake. In this case the amount of compound employed may be less than 1% by weight of the diet and in preferably no more than 0.5% by weight. The diet for animals may consist of normal foodstuffs to which the compound may be added or it may be added to a premix.

A suitable method of administration of a compound of formula (II) to animals is to add it to the animals drinking water. In this case a concentration of compound in the drinking water of about 5—500 $\mu$g/ml, for example 5—200 $\mu$g/ml, is suitable.

Compounds of formula (II) may be prepared by esterification of a carboxylic acid of formula (I) or a salt or other reactive derivative of the acid with an alcohol of formula ROH or an esterifying derivative thereof, wherein R is as defined with respect to formula (II).

A preferred process comprises reacting an alkali metal salt of the acid of formula (I), suitably the sodium salt, with a compound of formula:

R—X

in which R is as defined with respect to formula (II) and X is a good leaving group such as a halogen

atom or an alkyl or aryl sulphonate group, preferably a chlorine or bromine atom or a mesylate or tosylate group.

Esterification may be performed by any conventional method, for example by reaction of the free acid with the appropriate alcohol ROH in the presence of a catalyst such as a strong acid, dry hydrogen chloride or p-toluenesulphonic acid; or by reaction of a salt of the free acid:

a) with the compound of formula R—X as defined above in the presence of a polar aprotic solvent such as dimethylformamide or dimethylacetamide with or without a trace of hexamethylphosphoramide; or

b) by phase transfer catalysis methods with the compound of formula R—X in aqueous and/or organic solution in the presence of a quaternary ammonium bisulphate or halide, or benzyltrimethylammonium halide.

The formulation of compound (II) wherein Y represents

$$-CH=CH-CH_2-\overset{|}{C}H-$$

may also be carried out by conventional transesterification methods, for example reaction of a suitable ester of compound (I) with the appropriate alcohol ROH in the presence of a catalyst such as sodium salt of the alcohol, or dry hydrogen chloride, p-toluenesulphonic acid, or potassium cyanide.

Alternatively, the symmetrical or mixed anhydride derivative of the acid (I) may be reacted with an alcohol ROH, or alkali metal or alkaline earth metal alkoxide such as LiOR, NaOR, or MgClOR wherein R is defined with respect to formula (II). Suitable mixed anhydrides are alkoxyformic anhydrides, or anhydrides with, for example trimethyl acetic acid, thioacetic acid, diphenylacetic acid, benzoic acid, phosphorus acid (such as phosphoric or phosphorous acids), sulphuric acid or aliphatic or aromatic sulphonic acids such as *p*-toluenesulphonic acid). The mixed or symmetrical anhydrides may be generated *in situ*. For example, a mixed anhydride may be generated using isobutyl chloroformate or ethyl chloroformate. When a symmetrical anhydride is employed, the reaction may be carried out in the presence of 2,4-lutidine as catalyst.

The compound of the present invention may also be prepared from the intermediate keton of formula (IV) by any method known to convert a ketone into an $\alpha,\beta$-unsaturated ester. One such process comprises reacting a compound of formula (IV), wherein Y is as defined with respect to formula (I) in which the hydroxyl groups may be protected, with a compound of formula (V) or (VI):

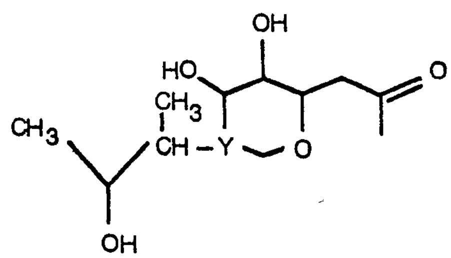

(IV)

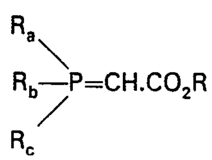

(VI)

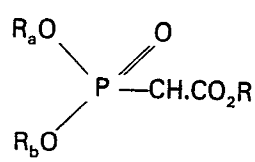

(V)

in which formulae (V) and (VI) the symbols $R_a$, $R_b$ and $R_c$ are the same or different and each is lower alkyl, aryl or aralkyl, and R is as defined with respect to formula (II) above; and subsequently removing any hydroxyl protecting groups.

The preferred embodiment of this process comprises reacting compound (IV) with compound (V). Preferably, in this case $R_a$ and $R_b$ are methyl or ethyl. In the case when compound (IV) is reacted with compound (VI), then $R_a$, $R_b$ and $R_c$ are preferably all phenyl.

The reaction is usually carried out in an inert solvent such as dimethylformamide, hexane, benzene, tetrahydrofuran for example, at a temperature of from about 10°C to about 100°C preferably under an inert gas such as nitrogen. Under these conditions the reaction proceeds smoothly over a period of from a few minutes to a few hours and the product may be isolated by any of the usual techniques, e.g. solvent evaporation or anti-solvent precipitation followed by filtration. In many cases the reaction may be carried out in a solvent in which the product is insoluble and in such cases the precipitation solid may be collected by filtration. Purification of the product may be by any of the usual chromatographic or recrystallisation techniques.

Compounds of formula (II) wherein Y represents

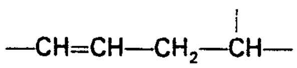

may also be prepared from compounds of formula (II) wherein Y represents

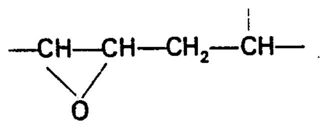

by reaction with a reagent which converts as epoxide to an olefin.

A number of reagents for converting an epoxide to an olefin are known in the literature, and the particular reagent of choice for the process of the present invention is a matter of trial and error. Some such reagents are more suitable than others for this purpose. Some generally applicable methods are as follows:

(a) Potassium selenocyanate in methanol/water; (see JCS Perkin I, 1975, 1216)

(b) Lower valent tungsten halides; for example $WCl_6$/butyl lithium (see J. Amer. Chem. Soc. 1972, *94*, 6538)

(c) $Ph_3P$ = Se/trifluoroacetic acid; (see JCS Chem. Comm. 1973, 253)

(d) Trifluoroacetyl iodide/sodium iodide; (see J. Org. Chem., 1978, *43*, 1841).

Other methods are described in the following references:

J. Amer. Chem. Soc., 1873, *95*, 2697.

Tet. Letts (17) 1976, 1385.

Ber. 1955, *88*, 1654.

J. Org. Chem., 1957, *22*, 1118.

It has been found that one convenient.method is the use of potassium selenocyanate.

Suitable solvents for use with potassium selenoxyanate include mixtures of water with alkanols, in particular $C_1$—$C_{20}$ alkanols. It has been found that higher yields of the compound of formula (II) are achieved if an alcohol is employed with a large, in particular branched or cyclic, alkyl group. Specific alcohols include *iso*-hexyl alcohol, *tert*-amyl alcohol and cyclohexyl alcohol. The reaction is generally performed at elevated temperatures, suitably at about the boiling point of the solvent employed. The time for which the reaction is performed depends on the temperature of the reaction, and therefore on the solvent. Generally a time of from 1—9 days is suitable.

Another suitable method for converting an epoxide into an olefin, comprises treatment with trifluoroacetyl iodide and sodium iodide. The trifluoroacetyl iodide may be prepared *in situ* from tirfluoroacetic anhydride. The reaction is suitably conducted at ambient temperatures for from about 10 to 36 hours, suitably about 24 hours.

Prior to the above processes of this invention, it may be desirable to protect the hydroxyl groups in compounds of formulae I and IV. Although reaction is possible without hydroxyl protection, in some cases higher yields could be formed if the hydroxyl groups were protected. Such protecting groups must be removable under suitably mild conditions and suitable groups include silyl groups produced from a silylating agent as discussed above. Particularly suitable hydroxyl-protecting groups include trimethylsilyl, t-butyldimethylsilyl, methylthiomethyl.

A preferred hydroxyl-protecting group is trimethylsilyl, as it is readily removed on completion of the reaction. Alternatively, for some reactions it is possible to protect the hydroxyl groups with other ester radicals which may be removed by chemical or enzymatic means. Examples include p-nitrobenzoate, methoxyacetate, phenoxyacetate, trifluoroacetate, each of which may be removed under mild basic conditions such as aqueous ammonia; or potassium carbonate in aqueous methanol.

It is also possible to protect the glcol moiety in compounds of formula I and suitable reagents for forming such a hydroxyl-protecting group include compounds of formula (VII).

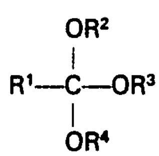

(VII)

wherein $R^1$ is hydrogen or a $C_{1-6}$ alkyl group and $R^2$, $R^3$ and $R^4$ independently represent a $C_{1-6}$ alkyl group.

The group $R^1$ may be for example hydrogen, methyl, ethyl, *n*- or *iso*-propyl. Most suitably, $R^1$ represents hydrogen so that the compound of formula (VII) is a trialkyl orthoformate.

Groups $R^2$, $R^3$ and $R^4$ may be for example, methyl, ethyl, *n*- or *iso*-propyl, *n*-, *iso*-, *sec*- or *tert*-butyl.

Preferably $R^2$, $R^3$ and $R^4$ are all the same and each represents a methyl group.

Other glycol protecting groups include those wherein the glycol moiety is converted to the structure:

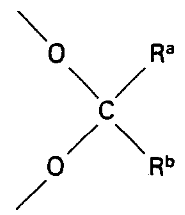

where $R^a$ and $R^b$ are hydrogen, $C_{1-6}$ alkyl, or phenyl. Preferably $R^a$ and $R^b$ are both methyl, i.e. the group is the isopropylidene group. This group may be introduced by reaction with 2,2-dimethoxypropane, and removed by treatment with acetic acid.

The hydroxy-protecting group may be removed by a conventional method for the particular hydroxyl-protecting group.

It may be such that it can be removed directly or alternatively, it may be converted into a different protecting group which is then removable under different conditions. This latter approach may be employed when a glycol protecting group derived from a compound (VII) is used; it is converted by acid to the group —$OCOR^1$ which is then removed.

The following Examples illustrate the preparation of a number of compounds of the present invention.

## Example 1

*Acetylmethyl Monate A*

Bromoacetone (0.411 g; 3 mM) and HMPA (1 drop) were added to a solution of sodium monate (1.098 g; 3 mM) in DMF (15 ml). This solution was stirred at room temperature for 16 hours. The solvent was then removed at reduced pressure and the residue partitioned between ethyl acetate and sodium bicarbonate solution. The organic layer was washed with saturated brine and dried over anhydrous $MgSO_4$. Removal of the solvent at reduced pressure gave a pale yellow oil (1.13 g). This oil was purified by silica gel column chromatography (Type 60; 15 g), eluting with 5% methanol/chloroform. The pure acetylmethyl monate A was obtained as a colourless oil (0.350 g; 20%), $\nu_{max}$ ($CHBr_3$) 3430, 1720 and 1642 $cm^{-1}$; $\lambda_{max}$ (EtOH) 222 nm ($\varepsilon$m 13,200); $\delta_H$ ($CDCl_3$) 5.85 (1H, s, CH=C), 4.63 (2H, s, $CH_2CO$), 2.18 (3H, s, $CH_3$—15), 2.14 (3H, s $COCH_3$) 1.19 (3H, d, J 6.5 Hz, $CH_3$—14), 0.91 (3H, d, J 6.5 Hz, $CH_3$—17); $\delta_C$ ($CDCl_3$) 203.0, 165.7, 159.8, 116.2, 75.0, 70.8, 70.4, 68.9, 67.9, 65.5, 61.0, 55.7, 42.8, 39.8, 31.8, 26.1, 20.7, 19.4 and 12.5; *m/e* (E.I.) 400.2077 (0.5%; $M^+$; $C_{20}H_{32}O_8$ requires 400.2059), 382 (1%), 365 (1), 227 (90), 111 (100), 43 (90).

## Example 2

*3-Acetylpropyl Monate A*

1-Methanesulphonoxy-3-acetylpropane (1.62 g, 9 mM) was dissolved in dry DMF (25 ml) with HMPA (1 drop). Sodium monate (2.196 g, 6 mM) was added and the solution heated at 80°C for 2 hours. The solvent was then removed at reduced pressure and the residue partitioned between ethyl acetate and sodium bicarbonate solution. The organic layer was washed with saturated brine and dried over anhydrous $MgSO_4$. Removal of the solvent at reduced pressure gave the ester as a pale yellow oil, which crystallised on scratching with ether. Digestion in ether/hexane gave the pure 3-acetylpropyl monate A as a white crystalline solid (0.749 g; 29%) mp 71—72°C, $\nu_{max}$ ($CHBr_3$) 3420, 1715 and 1643 $cm^{-1}$; $\lambda_{max}$ (EtOH) 220 nm ($\varepsilon$m 14,500); $\delta_H$ ($CDCl_3$) 5.73 (1H, s, CH=C), 4.08 (2H, t, $CO_2CH_2$), 2.52 (2H, t, $CH_2CO$), 2.18 (3H, s, $CH_3$—15), 2.14 (3H, s, $COCH_3$), 1.20 (3H, d, J 6.5 Hz, $CH_3$—14), 0.92 (3H, d, J 6.5 Hz, $CH_3$—17), $\delta_C$ ($CDCl_3$) 208.7, 166.8, 157.9, 117.2, 75.0, 70.4, 68.9, 65.4, 62.8, 60.7, 55.7, 49.9, 43.0, 42.6, 40.0, 31.8, 29.8, 23.0, 20.4, 19.1, 12.2. *m/e* (E.I.) 428.2419 (0.2%) ($M^+$; $C_{22}H_{36}O_8$ requires 428.2429), 410, 392, 366, 227 (50), 111 (40), 85 (100), 43 (100).

## Example 3

*8-Acetyloctyl Monate A*

Sodium monate (0.530 g; 1.3 mM; 90% pure) and HMPA (1 drop) were added to a solution of the mesylate of 8-acetyloctyl alcohol (1.30 mM) in dry DMF (10 ml). This solution was stirred at room temperature for 16 hours and 80°C for 2 hours, when tlc (9:1 $CHCl_3$/$CH_3OH$) indicated complete reaction. The solvent was then removed at reduced pressure and the residue partitioned between ethyl acetate and sodium bicarbonate solution. The organic layer was washed with saturated brine and dried over anhydrous $MgSO_4$. Removal of the solvent at reduced pressure gave a pale yellow oil which crystallised on standing. This material was digested in ether/hexane to give the pure 8-acetyloctyl monate A as a white crystalline solid (0.298 g; 46%) mp 69—71°C, $\nu_{max}$ ($CHBr_3$) 3420, 1700 and 1640 $cm^{-1}$; $\lambda_{max}$ (EtOH) 219 nm ($\varepsilon$m 15,400); $\delta_H$ ($CDCl_3$) 5.73 (1H, s, CH=C), 4.04 (2H, t, $CO_2CH_2$—),

2.40 (2H, t, $CH_2$—CO), 2.18 (3H, s, $CH_3$—15), 2.10 (3H, s, $COCH_3$), 1.3 (methylene envelope), 1.19 (3H, d, J 7.0 Hz, $CH_3$—14), 0.91 (3H, d, J 7.0, Hz, $CH_3$—17); $\delta_C$ ($CDCl_3$) 209.7, 166.9, 156.9, 117.6, 75.0, 71.0, 70.4, 68.9, 65.4, 63.8, 61.1, 55.6, 43.8, 42.8, 39.6, 31.7, 29.8, 29.2, 29.1, 28.7, 25.9, 23.8, 20.7, 19.1, 12.6; *m/e* (ammonia C.I.) 516 (30%; M + $NH_4^+$), 499 (65% M + $H^+$); *m/e* (E.I. 498 (1; $M^{+\cdot}$), 480.3062 (2; $M^+$—$H_2O$; $C_{27}H_{44}O_7$ requires 480.3037), 254 (55), 227 (90), 209 (20), 69 (100%).

Example 4

*4(1-Thienyl-2-ylbutan-1-only) monate A*

Sodium monate A (1 g) in dry DMF (25 ml) and HMPA (3 drops) was stirred with 4-chloro-1-(2-thienyl)butan-1-one (0.26 ml) and sodium iodide (0.5 g) for 16 hours at 80°C. After evaporation in vacuo, the residue was partitioned between ethyl acetate and water, and the organic phase washed with 10% sodium thiosulphate solution, saturated sodium bicarbonate solution and brine. The solution was dried ($MgSO_4$) and the solvent removed under reduced pressure to give an oil which was chromatographed on silica (type 60, 20 g) eluting with 0 to 2% methanol/chloroform. The pure product was obtained as a colourless oil (0.12 g 8%), $\nu_{max}$ (KBr) 3450 (broad), 2970, 1710 and 1665 $cm^{-1}$; $\lambda_{max}$ (EtOH) 220 ($\epsilon$m 15,200), 258 ($\epsilon$m 10,000) and 282 nm ($\epsilon$m 7,600); $\delta_H$ ($CD_3OD$) 7.85 (2H, m,

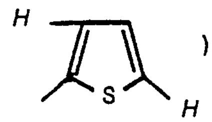
), 7.17 (1H, dd,
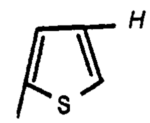
)

5.71 (1H, s, H—2), 4.12 (2H, t, $CO_2CH_2$), 3.02 (2H, t, $CH_2CO$), 2.13 (3H, s, $CH_3$—15), 1.18 (3H, d, $CH_3$—14) and 0.91 (3H, d, $CH_3$—17); $\delta_C$ ($CD_3OD$) 194.5 (Cl'), 168.1 (Cl), 159.3 (C3), 135.2, 133.9, 130.3, 129.4 C2"—5"), 118.1 (C2), 76.2 (C5, 71.6 (C13), 70.8 (C7), 70.0 (C6), 66.4 (C16), 64.0 (C4'), 61.3 (C11), 56.9 (C10), 43.9, 43.8 (C4, 12), 41.7 (C8), 36.5 (C2'), 33.0 (C9), 25.0 (C3'), 20.4 (C14), 19.3 (C15), 12.3 (C17); *m/e* (relative intensity) 496 ($M^+$, 1%), 227 (50), 153 (100), 111 (65), 69 (45) (found: $M^+$ 496.2148. $C_{25}H_{36}O_8S$ requires 496.2130).

Example 5

*4-Phenylbutan-4-only monate A*

A solution containing sodium monate A (1g, 2.7 mmol) 1-phenyl-4-chlorobutan-1-one (0.45 ml, 2.7 mmol), HMPA (1 drop), sodium iodide (0.5 g), and dry DMF (20 ml) was stirred for 16 hours at 80° with protection from moisture, and then evaporated *in vacuo* (40°C). The residue was taken up in ethyl acetate and the resulting mixture washed in turn with brine, aqueous sodium bicarbonate, and more brine, then dried ($MgSO_4$), and evaporated *in vacuo*. The resulting residue was purified by chromatography using type 60 silica gel (20 g) and 2% methanol in chloroform. This gave a colourless oil which was crystallised with ether to give product as a white powder m.p. 95°—97° (0.11 g, 8%, up to 14% on a larger scale, $\nu_{max}$ (KBr) 2900, 1710, 1670, 1645 $cm^{-1}$; $\lambda_{max}$ (EtOH) 231 nm ($\epsilon_m$ 17,900); $\delta_H$ ($CDCl_3$) 0.91 (3H, d, $CH_3$—17), 1.18 (3H, d, $CH_3$—14), 2.18 (3H, S, $CH_3$—15), 3.04 (2H, t, H—3'), 4.16 (2H, t, H—1'), 5.72 (1H, S, H—2), 7.4—7.6, 7.9—8.0 (5H, 2m, aryl); $\delta_c$ ($CDCl_3$), 12.7 (C17), 19.2 (C15), 20.8 (C14), 23.4 (C2'), 31.7 (C9), 35.1 (C3'), 39.6 (C8), 42.9 (C4, C12), 55.6 (C10), 61.3 (C11), 63.0 (C1'), 65.4 (C16), 69.0 (C6), 70.5 (C7), 71.3 (C13), 74.9 (C5), 117.4 (C2), 128.1, 128.6, 133.1, 137.0 (aryl), 157.3 (C3); $[\alpha]_D^{20}$ −8.3 (*C* = 1.0, $CHCl_3$); *m/e* (relative intensity) 490 ($M^+$, 1%), 227 (22), 147 (100), 105 (19) (Found = $M^+$ 490.2583. $C_{27}H_{38}O_8$ requires 490.2600); (Found: C 66.33, H 8.08. $C_{27}H_{38}O_8$ requires C 66.12, H 7.81%).

Example 6

*Cyclopentan-2-onyl monate A*

A solution containing sodium monate A (1 g, 2.7 mmol), 2-chlorocyclopentanone (0.34 ml, 2.7 mmol), HMPA (1 drop) sodium iodide (0.5 g), and dry DMF (20 ml) was stirred for 3 hours at 80° with protection from moisture, and then evaporated *in vacuo* (40°). The residue was taken up in ethyl acetate and the resulting mixture washed in turn with brine, aqueous sodium bicarbonate, and more brine, then dried ($MgSO_4$), and evaporated *in vacuo*. The resulting residue was purified by chromatography using type 60 silica gel (20 g) and 2% methanol in chloroform. This gave the product as a colourless oil (0.64 g, 56%), $\nu_{max}$ (film) 1705, 1660, 1640 $cm^{-1}$; $\lambda_{max}$ (EtOH) 224 nm ($\epsilon$M 12,600); $\delta_H$ ($CDCl_3$) 0.91 (3H, d, $CH_3$—17), 1.21 (3H, d, $CH_3$—14), 2.20 (3H, S, $CH_3$—15), 5.08 (1H, t, H—1'), 5.81 (1H, S, H—2); $\delta_c$ ($CDCl_3$) 12.2. (C17), 16.9 (C4'), 19.0, 19.2 (C15), 20.4 (C14), 28.3 (C3'), 31.4 (C9), 34.8 (C5'), 39.3 (C8), 42.4 (C12), 42.6, 42.8 (C4), 55.3 (C10), 60.8 (C11), 65.2 (C16), 68.5, 68.6 (C6), 70.0 (C7), 70.7 (C13), 74.6 (C1'), 74.8 (C5), 116.1, 116.2 (C2), 159.0 (C3), 165.3 (C1), 213.2 (C2'); *m/e* (relative intensity) 426 ($M^+$, 1%), 364 (5), 227 (56), 182 (66), 111 (100) (Found = $M^+$ 426.2263, $C_{22}H_{34}O_8$ requires 426.2276).

Example 7

*5-Acetylphenyl monate A*

7-Chloroheptan-2-one (0.45 g) and sodium iodide (0.45 g) were added to a solution of sodium monate A (1.1 g) in DMF (25 ml) and heated at 80°C overnight. The reaction was evaporated under reduced pressure and the residue dissolved in ethyl acetate/brine. The organic phase was washed with saturated sodium bicarbonate and brine, then dried ($MgSO_4$) and evaporated *in vacuo*. The product was chromatographed on silica (10 g) eluting with 0—4% methanol-chloroform, and fractions containing pure product (tlc) were combined and evaporated to give (670 mg, 49%), m.p. 62—4°C $\nu_{max}$ ($CHCl_3$) 3400 (broad) 1708 and 1645 $cm^{-1}$; $\lambda_{max}$ (EtOH) 222.5 nm ($\varepsilon$11,400); $\delta_H$ ($CDCl_3$) 0.92 (3H, d, $CH_3$—17), 1.22 (3H, d, $CH_3$—14), 2.10 (3H, s, $COCH_3$), 1.18 (3H, s, $CH_3$—15), 5.75 (1H, s, H—2); $\delta_C$($CDCl_3$) 208.5 (C6′), 166.8 (C1), 156.8 (C3), 117.9 (C2), 75.5 (C5), 71.2 (C13), 70.8 (C7), 69.3 (C6), 65.6 (C16), 63.6 (C1′), 61.2 (C11), 55.6 (C10), 43.6 (C5′), 43.1 (C4), 43.0 (C12), 39.9 (C8), 32.1 (C9), 29.7 (C2′), 28.8 (C3′), 25.9 (C4′), 23.7 (C7′), 20.9 (C14), 19.4 (C15),12.6 (C17); *m/e* (relative intensity) 456 (1), 538 (1), 227 (45), 43 (100). (Found: 456.2703. $C_{24}H_{40}O_8$ requires 456.2686).

Example 8

*Cycohexan-4-onyl monate A*

To a solution of 4-hydroxycyclohexanone (4.0 g, 35 mmol) in dichloromethane (50 ml) containing triethylamine (6.3 ml, 45 mmol) was added, at −10°, mesyl chloride (3.1 ml, 40 mmol) during 10 min. The mixture was stirred at −10° for 20 min and then poured into cold water. The organic layer was washed with cold aqueous hydrochloric acid, cold aqueous sodium bicarbonate, and brine, dried ($MgSO_4$) and evaporated *in vacuo* to leave 4-mesyloxycyclohexanone as an oil (6.1 g, 91%). (Any contaminating cyclohexa-1,4-dione is mostly removed during this procedure).

$\nu_{max}$ (film 1715, 1350, 1175 $cm^{-1}$; $\delta_H$ ($CDCl_3$) 5.1 (1H, m, H4), 3.05 (3H, s, $CH_3$), 2.0—2.8 (8H, m, *$CH_2$*).

A mixture of sodium monate A (5.5 g, 0.015 mol), 4-mesyloxycyclohexanone (3.0 g, 0.015 mol), sodium iodide (2.5 g) and DMF (100 ml) was stirred together at 60° for 16 h, then evaporated *in vacuo* and the residue dissolved in ethyl acetate. The resulting solution was washed with brine, aqueous sodium bicarbonate, and more brine, dried ($MgSO_4$), and evaporated *in vacuo*. Purification of the residue by chromatography on silica gel (30 g), eluting with 0—4% methanol in chloroform, gave the pure keto-ester as a colourless oil (0.56 g, 9%), $\nu_{max}$ (film) 3450, 1710, 1645, 735 $cm^{-1}$; $\lambda_{max}$ (EtOH) 223 nm ($\varepsilon$m 11,900); $\delta$H ($CDCl_3$) 5.82 (1H, s, H2), 5.22 (1H, m, H1′), 2.21 (3H, s, $CH_3$—15), 1.18 (3H, d, $CH_3$—14), 0.91 (3H, d, $CH_3$—17); $\delta$C ($CDCl_3$) 210.4 (C4′), 165.9 (C1), 157.8 (C3), 117.5 (C2),75.0 (C5), 71.3 (C13), 70.5 (C7), 69.1 (C6), 67.8 (C1′), 65.5 (C16), 61.3 (C11), 55.6 (C10), 43.0 (C4), 42.9 (C12), 39.7 (C8), 37.4 (C3′, C5′), 31.7 (C9), 30.6 (C2′, C6′), 20.8 (C14), 19.3 (C15), 12.7 (C17); *m/e* (relative intensity) 440 ($M^+$, 1%), 378 (6), 227 (81), 97 (100) (Found: $M^+$ = 440.2412, $C_{23}H_{36}O_8$ requires 440.2414).

Example 9

*4-(4-Acetamidophenyl)-4-oxobutyl monate A*

A mixture of carbon disulphide (110 ml), 4-chlorobutanoyl chloride (28 ml, 0.25 mol), and acetanilide (20 g, 0.15 mol) was stirred at reflux during the careful addition of aluminium chloride (60 g, 0.45 mol) over 20 min, and then for a further 40 min. The mixture was then cooled, the upper layer discarded, and the lower (black) layer decomposed with a mixture of ice (200 g) and concentrated hydrochloric acid (10 ml). Filtration and trituration of the residue with methanol gave the crude ketone as a brown powder which was recrystallised from ethanol to give fawn crystals of N-(4-(4-chloro-1-oxobutyl)phenyl)-acetamide, m.p. 160° (7.2 g, 20%) $\nu_{max}$ (KBr) 1680, 1600, 1540, 830 $cm^{-1}$; $\lambda_{max}$ (EtOH) 219 nm ($\varepsilon$m 10,600), 287 nm ($\varepsilon$m 19,500); $\delta$H ($CDCl_3$/DMSO-$d_6$) 9.6 (1H, bs, N*H*), 7.8 (4H, ABq, aryl), 3.6 (2H, t, H4), 3.05 (2H, t, H2), 2.15 (2H, m, H3), 2.1 (3H, s, $CH_3$); (Found C 59.89, H 5.84, N 5.86%: $C_{12}H_{14}ClNO_2$ requires C 60.13, H 5.89, N 5.86%).

A mixture of sodium monate A (1.1 g, 3 mmol), N-(4-(4-chloro-1-oxobutyl)phenyl)-acetamide (0.7 g, 3 mmol), DMF (25 ml), sodium iodide (0.5 g) and HMPA (1 drop) were stirred together at 80° for 6 h. The mixture was then evaporated *in vacuo* and taken up in ethyl acetate, whcih was then washed with brine, aqueous sodium bicarbonate, and more brine, and then dried ($MgSO_4$) and evaporated *in vacuo*. Purification of the residue by chromatography on silica gel (10 g), eluting with chloroform containing 0—4% methanol, gave pure ester as a yellow oil (130 mg, 8%) $\nu_{max}$ (film) 3400. 1675, 1590, 1530 $cm^{-1}$; $\lambda_{max}$ (EtOH) 219 nm ($\varepsilon$m 21,500), 285 nm ($\varepsilon$m 17,600); $\delta$H ($CDCl_3$) 9.25 (1H, s, NH), 7.76 (4H, ABq, aryl), 5.72 (1H, s, H2), 4.18 (2H, t, H1′), 2.21 (3H, s, $CH_3$—15), 1.21 (3H, d, $CH_3$—14), 0.91 (3H, d, $CH_3$—17); *m/e* (relative intensity) 547 ($M^+$, 1%), 227 (45), 204 (100) (Found: $M^+$ = 547.2753, $C_{29}H_{41}NO_9$ requires 547.2727).

Example 10

*Cyclooctan-5-onyl monate A*

A solution of *cis*-1,5-cycloöctanediol (2.15 g, 0.015 mol) in pyridine (10 ml) was kept at 0° during the addition of a solution of toluenesulphonyl chloride (2.85 g, 0.015 mol) in chloroform (10 ml);

and then stood at 20° overnight, washed with water, dilute hydrochloric acid, and then brine, dried ($MgSO_4$) and evaporated *in vacuo* to give *cis*-5-tosyloxycyclooöctanol as a colourless oil (3.3 g, 74%), i.r. (film) 3320b, 1600, 1175 $cm^{-1}$; $\delta_H$ ($CDCl_3$) 7.2—7.4, 7.7—7.9 (4H, ABq, aryl), 4.6 (1H, bs, H5), 3.7 (1H, bs, HI), 2.4 (3H, s, Me), 1.3—2.0 (12H, m, $CH_2$).

A mixture of *cis*-5-tosyloxycyclooöctanol (9.0 g, 0.030 mol), pyridinium chlorochromate (7.5 g, 0.035 mol) and dichloromethane was allowed to stand for 16 h at 5° and then diluted with ether and filtered. The filtrate was evaporated *in vacuo* and the resulting residue extracted with benzene/petrol. Evaporation in turn of these extracts gave a brown oil which could be recrystallised from ether to give the ketone as white crystals, m.p. 67°—70° (3.5 g, 39%), i.r. (film) 1695, 1595, 1175 $cm^{-1}$, $\delta_H$ ($CDCl_3$ 7.2—7.4, 7.6—7.8 (4H, ABq, aryl), 4.3 (1H, bs, *CH*), 2.4 (3H, s, Me), 1.5—2.0, 2.1—2.5 (12H, 2m, $CH_2$); (Found: C 60.94, H 6.40, $C_{15}H_{20}O_4S$ requires C 60.79, H 6.80).

A solution containing sodium monate A (1.0 g, 2.7 mmol), 5-tosyloxycylooöctanone (0.8 g, 2.7mmol), HMPA (1 drop), sodium iodide (0.5 g) and DMF (25 ml) was stirred at 80° for 16 h, and then evaporated *in vacuo*. The residue was dissolved in ethyl acetate and the resulting solution was washed with aqueous sodium bicarbonate, then brine, dried ($MgSO_4$) and evaporated *in vacuo*. Purification of the residue by column chromatography (20 g silica gel, 0 to 4% methanol in chloroform) then gave pure ester as a colourless oil (0.18 g, 14%), i.r. (film) 3440b, 1700, 1645, 755 $cm^{-1}$; UV (EtOH) $\lambda_{max}$ 222 nm ($\varepsilon_m$ 13,9000); $\delta_H$ ($CDCl_3$ 5.68 (1H, s, H2), 4.70 (1H, quin, H1'), 2.19 (3H, s, $CH_3$—15), 1.21 (3H, d, $CH_3$—14), 0.94 (3H, d, $CH_3$—17); *m/e* (relative intensity) 468 ($M^+$, 1%), 227 (43), 55 (100) (Found $M^+$ = 468.2722, $C_{25}H_{40}O_8$ requires 468.2723).

*Biological Data*

a) *Human Bacteria*

Table 1 shows the MIC values (μg/ml) of the compounds of Examples 1 to 10 against a number of organisms important in human infections obtained by serial dilution in nutrient agar containing 5% 'chocolated' horse blood.

TABLE 1

| Organism | Compound of Example No. 1 | 2 | 3 | 4 | 5 | 6 | 7 | 8 | 9 | 10 |
|---|---|---|---|---|---|---|---|---|---|---|
| E coli NCTC 10418 | 100 | 100 | 100 | >100 | >100 | >100 | >100 | >100 | >100 | >100 |
| E coli ESS | 50 | 0.5 | 0.5 | >100 | 1.0 | 2.5 | 0.5 | 0.5 | 1.0 | 2.5 |
| P mirabilis 889 | >100 | 25 | >100 | >100 | >100 | 100 | >100 | 100 | >100 | >100 |
| K aerogenes A | >100 | 100 | >100 | >100 | >100 | >100 | >100 | >100 | >100 | >100 |
| Ps aeruginosa 10662 | >100 | >100 | >100 | >100 | >100 | >100 | >100 | >100 | >100 | >100 |
| Pasteurella multocida 1633 | 10 | 0.2 | 1.0 | 1.0 | 1.0 | 1.0 | 0.5 | 0.5 | 1.0 | 2.5 |
| Haemophilus influenzae Q1 | 2.5 | 0.05 | 0.2 | 0.2 | — | — | 0.2 | — | — | 0.5 |
| Haemophilus influenzae Wy21 | 2.5 | 0.05 | 0.2 | 0.2 | 0.1 | 0.5 | 0.1 | 0.1 | 0.25 | — |
| Neisseria flavescens 8263 | — | 0.1 | 0.1 | 0.2 | [a]0.2 | [a]1.0 | [a]0.5 | [a]0.5 | [a]0.5 | [a]2.5 |
| Bacillus subtilis 6633 | 10 | 0.2 | 0.2 | 0.2 | 0.1 | 2.5 | 0.5 | 0.5 | 0.25 | 1.0 |
| Corynebacterium zerosis 9755 | >100 | 100 | 100 | >100 | >100 | >100 | | >100 | >100 | >100 |
| Sercina lutea 8340 | >100 | 100 | >100 | 50 | >100 | >100 | ″ | — | — | >100 |
| Staph aureus Oxford | 10 | 0.2 | 0.1 | 1.0 | 0.5 | 2.5 | 1.0 | 1.0 | 0.5 | 1.0 |
| Staph aureus Russell | 25 | 1.0 | 9.5 | 2.5 | 0.5 | 5.0 | 1.0 | 2.5 | 1.0 | 2.5 |
| Staph aureus W2827 | 25 | 1.0 | 100 | 1.0 | 0.5 | 5.0 | 2.5 | 2.5 | 1.0 | 2.5 |
| Strep faecalis I | >100 | >100 | 100 | 50 | 50.0 | >100 | >100 | 25.0 | 50 | 100 |
| Strep pyogenes A 64/848 | 25 | 1.0 | 0.2 | 0.5 | 0.5 | 2.5 | 0.5 | [b]0.5 | [b]0.25 | [b]1.0 |
| Strep pyogenes B 2788 | 25 | 1.0 | 0.5 | 2.5 | 0.5 | 5.0 | 2.5 | 0.5 | 0.25 | 2.5 |
| Strep pyogenes C 2761 | 25 | 1.0 | 0.5 | 1.0 | 0.5 | 5.0 | 2.5 | [c]0.5 | [c]0.25 | [c]2.5 |
| Strep pneumoniae CN33 | 25 | 0.5 | 0.2 | 1.0 | — | — | 0.5 | 0.5 | 0.25 | 2.5 |

[a]Neisseria catarrhalis 1502 [b]Strep pyogenes A R80/421 [c]Strep pyogenes C 641848

b) *Veterinary Bacteria*

Table 2 shows the MIC values (μg/ml) of the compounds of the Examples against a number of organisms important in veterinary infections.

TABLE 2

| Organism | Compounds of Example No. 1 | 2 | 3 | 4 | 5 | 6 | 7 | 8 | 9 | 10 |
|---|---|---|---|---|---|---|---|---|---|---|
| E coli NCTC 10418 | >80 | >80 | >80 | >80 | 80 | >80 | 80 | >80 | >80 | >80 |
| E coli E1 | >80 | >80 | >80 | >80 | 80 | >80 | >80 | >80 | >80 | >80 |
| S dublin S7 | >80 | >80 | >80 | >80 | >80 | >80 | >80 | >80 | >80 | >80 |
| S typhimurium S18 | >80 | >80 | >80 | >80 | 80 | >80 | >80 | >80 | >80 | >80 |
| Bord bronchiseptica BO8 | >80 | 40 | 20 | 40 | 40 | 80 | 40 | 80 | >80 | >80 |
| Bord bronchisptica B09 | 40 | 5 | 5 | 5 | 5 | 20 | 5 | 20 | 20 | 80 |
| Past multocida PA1 | 0.625 | 0.312 | 2.5 | 0.312 | 1.25 | .625 | .625 | .312 | 2.5 | 40 |
| Past multocida PA2 | 0.312 | 0.039 | 0.156 | 0.07 | .312 | .312 | .312 | .156 | .625 | 1.25 |
| Past Haemolytica PA5 | 5 | 5 | 20 | 5 | 10 | 5 | 5 | 5 | 20 | 40 |
| Erysipelothrix rhusiopathiae NCTC 8163 | >80 | 80 | 10 | 20 | 40 | >80 | 40 | 10 | 20 | 80 |
| Corynebacterium pyogenes CY1 | >80 | — | >80 | >80 | >80 | >80 | >80 | >80 | >80 | 80 |
| Staph aureus B4 (pen resis) | 2.5 | 1.25 | 0.312 | 0.156 | .156 | 2.5 | .625 | 2.5 | 1.25 | 2.5 |
| Staph aureus 152 (pen sens) | 2.5 | 1.25 | 0.312 | 0.156 | .156 | 2.5 | .625 | 1.25 | .625 | 2.5 |
| Staph aureus Oxford | 2.5 | 1.25 | 0.312 | 0.156 | .156 | 2.5 | .625 | 1.25 | .625 | 2.5 |
| Strep suis (group D) SPS11 | 40 | 10 | 1.25 | 2.5 | 5 | 20 | 5 | 2.5 | 2.5 | 80 |
| Strep uberis SPU1 | 2.5 | 0.312 | 0.07 | <0.019 | .07 | 1.25 | .156 | .312 | .156 | 5 |
| Strep dysgalaciae SPD1 | 5 | 1.25 | 0.156 | 0.516 | .07 | 1.25 | .312 | .156 | .156 | 5 |
| Strep agalactiae SPA1 | 5 | 2.5 | 0.625 | 0.312 | .625 | 2.5 | .625 | .625 | .625 | 10 |

c) *Anti-Mycoplasmal Activity*

Table 3 shows the *in vitro* MIC values (μg/ml) of the compounds of Examples 1 to 4 against a number of mycoplasma organisms.

Table 4 shows the *in vitro* MIC values (μg/ml) of the compounds of Examples 5 to 10 against a number of mycoplasma organisms. The values were determined in Friis broth solidified with 0.9% agarose. The inoculum was $10^3$ to $10^5$ C.F.U. and the MIC's were recorded after 6 days incubation at 37°C.

TABLE 3

| Organism | Compound of Example No. 1 | | 2 | | 3 | | 4 |
|---|---|---|---|---|---|---|---|
| | BROTH* | AGAR+ | BROTH* | AGAR+ | BROTH* | AGAR+ | BROTH* |
| M suipneumoniae Str 11 | 0.625 | | 0.156 | | 0.039 | | 0.078 |
| M suipneumoniae J2206/183b | 1.25 | | 0.312 | | 0.156 | | 0.078 |
| M dispar H225 | 0.156 | | ⩽0.02 | | 0.039 | | <0.02 |
| M dispar NCTC 10125 | 0.156 | | ⩽0.02 | | <0.02 | | 0.039 |
| M pneumoniae 427a | >10 | 10 | 0.625 | 0.312 | 0.312 | 1.25 | 0.312 |
| M pneumoniae ATCC 15492 | >10 | >10 | 0.312 | 0.312 | 0.312 | 1.25 | 0.312 |
| M bovis ATCC 25025 | | 0.078 | | <0.02 | | 0.039 | |
| M bovis NCTC 10131 | | 0.078 | | <0.02 | | ⩽0.02 | |
| M fermentans MWKL4 | 0.078 | 0.312 | <0.02 | ⩽0.02 | <0.02 | 0.039 | <0.02 |
| M pulmonis JB | 0.156 | 0.156 | <0.02 | ⩽0.02 | <0.02 | 0.039 | <0.02 |
| M hyorhinis ATCC 23234 | | 2.5 | | 0.312 | | 0.625 | |
| M hyosynoviae ATCC 25591 | | 1.25 | | 0.078 | | 0.156 | |
| M arthritidis ATCC 14124 | | >10 | | 2.5 | | 2.5 | |
| M gallisepticum S6 | | >10 | | 2.5 | | 5.0 | |
| M synoviae ATCC 25204 | | <0.02 | | <0.02 | | <0.02 | |
| M alkalescens NCTC 10135 | | 0.039 | | <0.02 | | 0.039 | |
| M bovigenitalium ATCC 14173 | | 0.078 | | ⩽0.02 | | 0.078 | |

* Determined in Friis' broth using microtiter method
\+ Determined by serial dilution in Friis' agar

TABLE 4

| Organism | Compound of Example No. 5 | 6 | 7 | 8 | 9 | 10 |
|---|---|---|---|---|---|---|
| M. suipneumoniae NB12 | 0.5 | 2.5 | 0.25 | 0.25 | 0.5 | 2.5 |
| M. suipneumoniae JF 435 | 0.5 | 10 | 0.25 | 0.5 | 0.5 | 2.5 |
| M. suipneumonia HK (2) | 0.5 | 10 | 0.5 | 0.5 | 0.5 | 2.5 |
| M. suipneumoniae Str. 11 | 0.25 | 2.5 | 0.25 | 0.25 | 0.25 | 1.0 |
| M. suipneumoniae J2206/183b | 0.5 | 10 | 0.5 | 0.5 | 0.5 | 2.5 |
| M. suipneumoniae MS 16 | 0.25 | 2.5 | 0.25 | 0.25 | 0.25 | 1.0 |
| M. suipneumoniae PW/C/210 | 0.25 | 2.5 | 0.25 | 0.25 | 0.25 | 1.0 |
| M. suipneumoniae LABER | 0.25 | 2.5 | 0.25 | 0.25 | 0.25 | 1.0 |
| M. suipneumoniae UCD 1 | 0.5 | 10 | 0.5 | 0.5 | 0.5 | 1.0 |
| M. suipneumoniae TAM 6N | 0.5 | 5.0 | 0.5 | 0.5 | 0.5 | 2.5 |
| M. suipneumoniae ATCC 25095 | 0.25 | 5.0 | 0.25 | 0.25 | 0.5 | 1.0 |
| M. suipneumoniae NCTC 10110 | 0.5 | 10 | 0.5 | 0.5 | 0.5 | 1.0 |
| M. hyorhinis ATCC 23234 | 0.25 | 2.5 | 0.1 | 0.25 | 0.25 | 1.0 |
| M. hyorhinis ATCC 25021 | 0.25 | 2.5 | 0.1 | 0.25 | 0.25 | 1.0 |
| M. hyosynoviae ATCC 25591 | 0.05 | 2.5 | 0.025 | 0.1 | 0.1 | 0.25 |
| M. bovis NCTC 10131 | $\leqslant$0.01 | 0.25 | <0.01 | 0.025 | $\leqslant$0.01 | 0.025 |
| M. bovigenitalium ATCC 14173 | 0.025 | 0.25 | 0.025 | 0.025 | 0.05 | 0.05 |
| M. dispar NCTC 10125 | 0.1 | 0.5 | 0.05 | 0.1 | 0.1 | 0.5 |
| M. gallisepticum S6 | 1.0 | >10 | 2.5 | 5.0 | 2.5 | >10 |
| M. pneumoniae ATCC 15492 | 1.0 | 10 | 0.5 | 1.0 | 0.5 | 10 |

d) *Serum Binding*

Serum binding was assessed by ultrafiltration of procine serum containing compounds at 8 mcg/ml, and through an Amicon CV50A ultrafiltration cone. Separation of ultrafiltrate was achieved by centrifugation. Unbound concentrations of compound were measured in the ultrafiltrate by microbiological assay (B. subtilis ATCC 6633) against standards prepared in saline.

| Example No. | % bound to pig serum |
|---|---|
| 1 | 22.8 |
| 2 | 24.0 |
| 3 | 97.3 |
| 4 | 98.8 |
| 5 | 96.4 |
| 6 | 35.6 |
| 7 | 61.5 |
| 8 | 34.5 |
| 9 | 86.2 |
| 10 | — |

e) *Mean serum concentration in neonatal piglets of Examples 4 and 5 after intramuscular or oral administration at 50 mg/kg*

Blood levels were assessed in neonatal piglets (2 to 4 animals per group and man bodyweights about 2 kg). The dose was 50 mg/kg (dose solution = 25 mg/ml in 25% ethanol). Doses were given by intramuscular injection and orally by stomach tube. Piglets were bled at intervals up to 8 and at 24 hours, and serum assayed microbiologically (*B. subtilis* ATCC 6633).

| Example No. | Route of Administration | Serum concentration (mcg/ml) at: | | | | | | | | | |
|---|---|---|---|---|---|---|---|---|---|---|---|
| | | 10′ | 20′ | 40′ | 60′ | 2h | 3h | 4h | 6h | 8h | 24h |
| 4 | i.m. | 9.0 | 8.9 | 10.0 | 8.5 | 3.3 | 1.9 | 1.6 | 0.83 | 0.71 | <0.12 |
| | p.o. | 5.3 | 5.5 | 6.5 | 5.7 | 2.4 | 1.4 | 1.4 | 0.77 | 0.74 | 0.15 |
| 5 | i.m. | 1.9 | 2.3 | 1.5 | 1.2 | 0.57 | 0.38 | 0.33 | 0.31 | 0.18 | <0.2 |
| | p.o. | 1.2 | 1.2 | 1.2 | 0.86 | 0.23 | 0.16 | 0.08 | 0.05 | 0.03 | <0.2 |

## Claims

1. A compound of the formula (II):

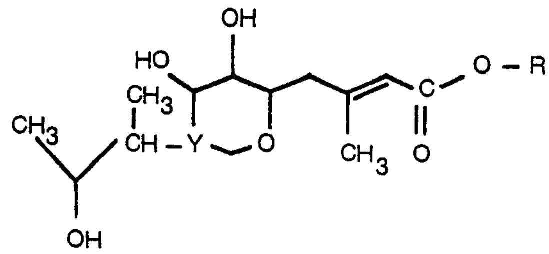

(II)

wherein Y represents 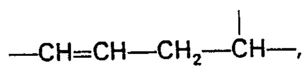, 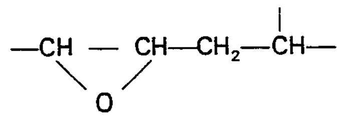 or

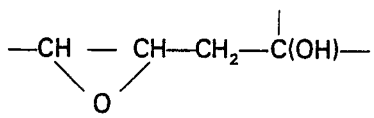

and R represents a $C_{3-20}$ alkyl, $C_{3-8}$ cycloalkyl, $C_{4-20}$ alkenyl, aralkyl, cycloalkylalkyl, heterocyclyl or heterocyclylalkyl group, each of which is substituted with a ketonic oxo group.

2. A compound according to claim 1, in which R represents a $C_{3-10}$ alkyl group, a heterocyclylalkyl group, or a $C_{5-8}$ cycloalkyl group, each of which is substituted with a ketonic oxo group.

3. A compound according to claim 1, of the formula (III):

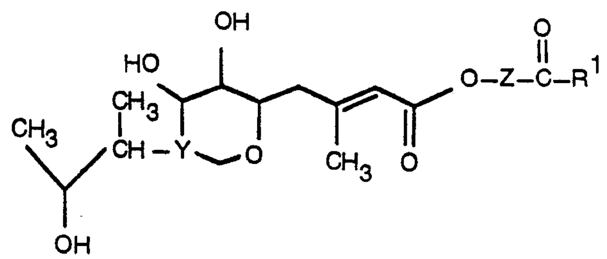

(III)

wherein Y is as defined with respect to formula (II), Z represents straight or branched chain $C_{1-12}$ alkylene; and $R^1$ represents a $C_{1-10}$ alkyl, $C_{3-8}$ cycloalkyl, $C_{2-10}$ alkenyl, aryl, aralkyl, cycloalkylalkyl or heterocyclyl group.

4. A compound according to claim 3, in which $R^1$ represents a $C_{1-8}$ alkyl, heterocyclyl or optionally substituted phenyl group and Z represents a straight chain $C_{1-8}$ alkylene group.

5. A compound according to any one of claims 1 to 4 in which Y represents a

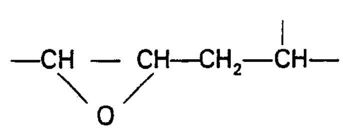

group.

6. A pharmaceutical or veterinary composition comprising a compound according to any one of claims 1 to 5 together with a pharmaceutically or veterinary acceptable carrier or excipient.

7. A composition according to claim 6 in dosage unit form.

8. A composition according to claim 6 in the form of an animal foodstuff or drinking water supply.

9. A process for the preparation of a compound of formula (II) as defined in claim 1, which comprises treating an acid of formula (I):

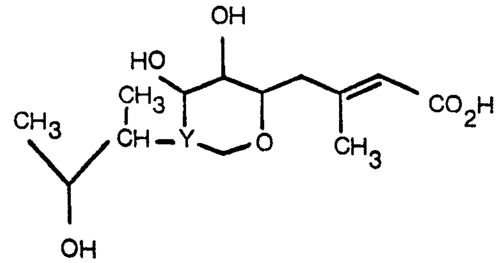

(I)

wherein Y represents

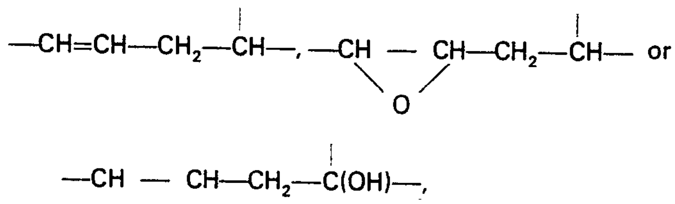

or a salt or reactive derivative thereof, with an alcohol of formula ROH, wherein R is as defined with respect to formula (II), or an esterifying derivative thereof.

10. A process according to claim 9 in which an alkali metal salt of the acid of formula (I) is reacted with an esterifying derivative of ROH having the formula R—X, in which X is a halogen atom, or an alkyl or aryl sulphonate group.

11. A process for the preparation of a compound of formula (II) as defined in claim 1, which comprises treating a symmetrical or mixed anhydride derivative of an acid of formula (I):

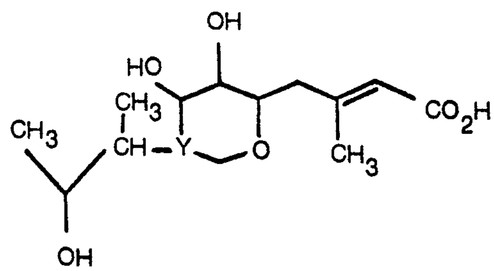

(I)

with an alcohol of formula ROH, or an alkali metal or alkaline earth metal alkoxide of the alcohol, wherein R is as defined with respect to formula (II).

12. A process for the preparation of a compound of formula (II) as defined in claim 1, which comprises reacting a compound of formula (IV):

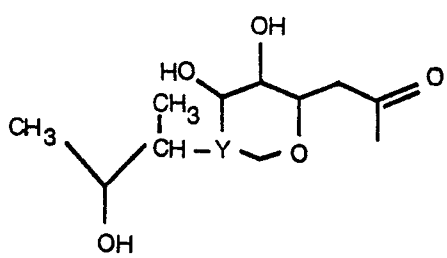

(IV)

wherein Y is as defined with respect to formula (II), with a compound of formula (V) or (VI):

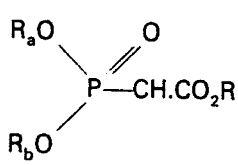

(V)

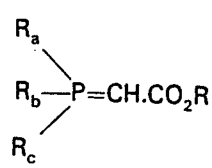

(VI)

wherein $R_a$, $R_b$ and $R_c$ are the same or different and each is lower alkyl, aryl or aralkyl, and R is as defined with respect to formula (II).

13. A process according to any one of claims 9 to 12, in which the hydroxyl groups in the compounds of formula (I) and (IV) are initially protected and are subsequently removed.

**Revendications**

1. Composé de formula (II):

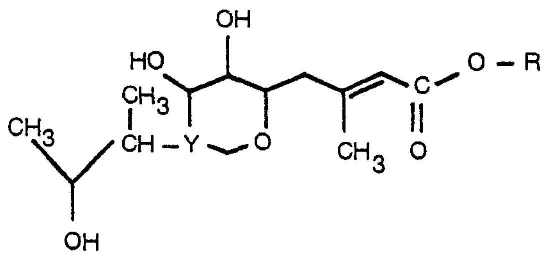

(II)

dans laquelle Y représente 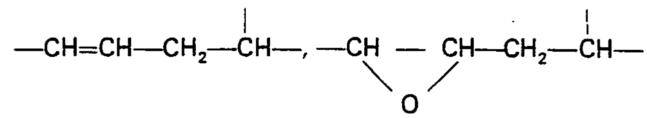 ou

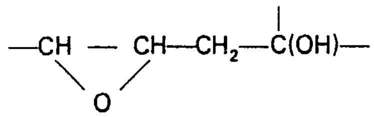

et R représente un groupe alcoyle en $C_{3-20}$, cycloalcoyle en $C_{3-8}$, alcényle en $C_{4-20}$, aralcoyle, cycloalcoylalcoyle, hétérocyclyle ou hétérocyclylalcoyle, dont chacun est substitué avec un groupe oxo cétonique.

2. Composé suivant la revendication 1, caractérisé en ce que R représente un group alcoyle en $C_{3-10}$, un groupe hétérocycylalcoyle ou un groupe cycloalcoyle en $C_{5-8}$, dont chacun est substitué par un groupe oxo cétonique.

3. Composé suivant la revendication 1, de formule (III):

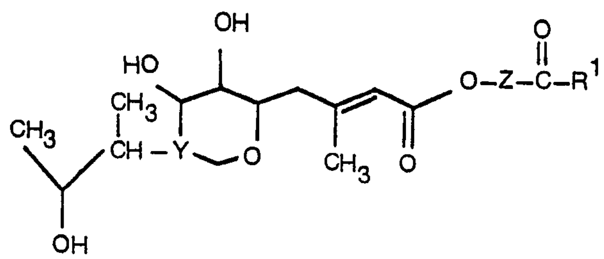

(III)

dans laquelle Y a la même signification que dans le cas de la formule (II), Z représente un alcoylène en $C_{1-12}$ à chaîne linéaire ou ramifiée; et $R^1$ représente un groupe alcoyle en $C_{1-10}$, cycloalcoyle en $C_{3-8}$, alcényle en $C_{2-10}$, aryle, aralcoyle, cycloalcoylalcoyle ou hétérocyclyle.

4. Composé suivant la revendication 3, caractérisé en ce que $R^1$ représente un groupe alcoyle en $C_{1-8}$, hétérocyclyle ou phényle facultativement substitué, et Z représente un groupe alcoylène en $C_{1-8}$, à chaîne linéaire.

5. Composé suivant l'une quelconque des revendications 1 à 4, caractérisé en ce que Y représente un groupe

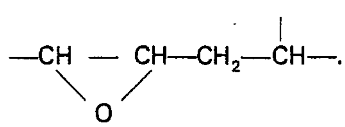

6. Composition pharmaceutique ou vétérinaire renfermant un composé suivant l'une quelconque des revendications 1 à 5 conjointement à un véhicule ou excipient pharmaceutiquement acceptable ou bien acceptable en médecine vetérinaire.

7. Composition suivant la revendication 6, sous forme posologique unitaire.

8. Composition suivant la revendication 6, sous forme d'une addition à la nourriture ou à l'eau de boisson des animaux

9. Procédé pour la préparation d'un composé de formule (II) tel que défini dans la revendication 1, consistant à traiter un acide de formule (I):

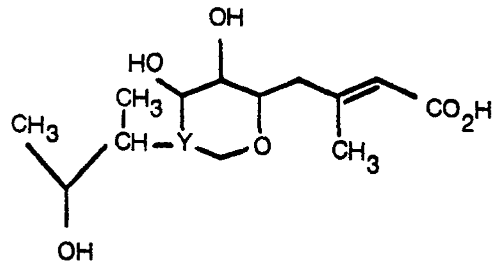

(I)

dans laquelle Y représente 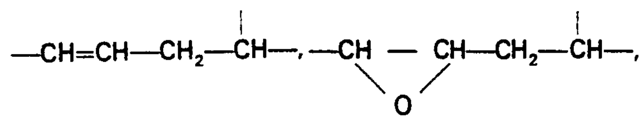 ou

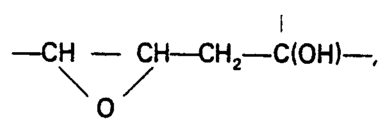

ou un sel ou dérivé réactif de celui-ci avec un alcool de formule ROH où R a la même signification que dans le cas de la formule (II), ou bien avec un dérivé d'esterification de celui-ci.

10. Procédé suivant la revendication 9, caractérisé en ce qu'on fait réagir un sel de métal alcalin de l'acide de formule (I) avec un dérivé d'estérification de ROH de formule R—X dans laquelle X est un atome d'halogène ou un groupe alcoyl- ou aryl-sulfonate.

11. Procédé pour la préparation d'un composé de formule (II) suivant la revendication 1, consistant à traiter un dérivé formé par un anhydride symétrique ou mixte d'un acide de formule (I):

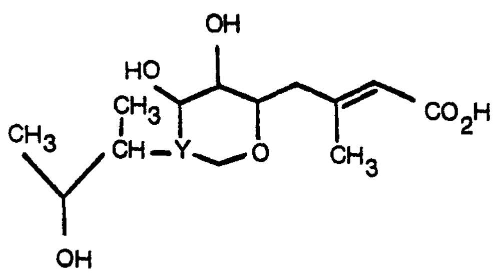

(I)

avec un alcool de formule ROH ou un alcoolate de métal alcalin ou de métal alcalino-terreux de l'alcool, formule dans laquelle R a la même signification que dans le cas de la formule (II).

12. Procédé pour la préparation d'un composé de formule (II) suivant la revendication 1, consistant à faire réagir un composé de formule (IV):

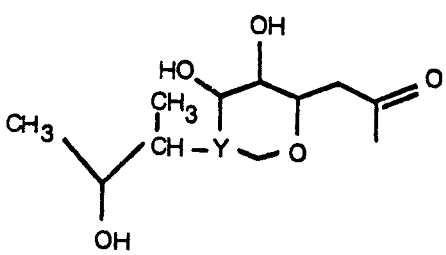

(IV)

dans laquelle Y a la même signification que dans le cas de la formule (II) avec un composé de formule (V) ou (VI):

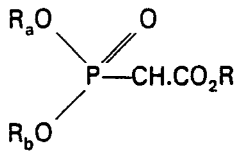

(V)

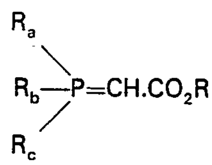

(VI)

das lesquelles $R_a$, $R_b$ et $R_c$ sont identiques ou différents et désignent chacun un groupe alcoyle inférieur, aryle ou aralcoyle, et R a la même signification que dans le cas de la formule (II).

13. Procédé suivant l'une quelconque des revendications 9 à 12, caractérisé en ce que les groupes hydroxy des composés de formules (I) et (IV) sont initialement protégés, les protections étant ensuite éliminées.

**Patentansprüche**

1. Eine Verbindung der Formel (II):

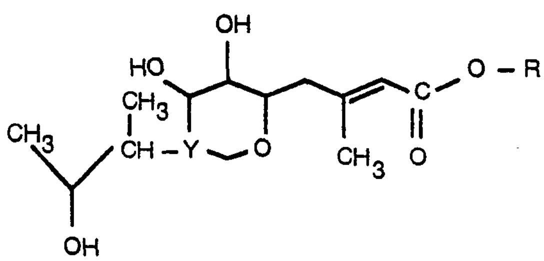

(II)

in der Y 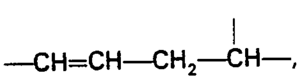, 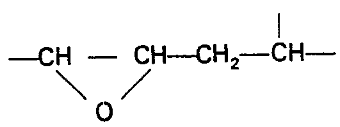 oder

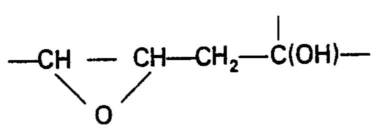

darstellt, und R eine $C_{3-20}$-Alkyl-, $C_{3-8}$-Cycloalkyl- $C_{4-20}$-Alkenyl, Arylalkyl-,. Cycloalkylalkyl-, Heterocyclyl- oder Heterocyclylalkyl-Gruppe bedeutet, wobei jede mit einer ketonische Oxogruppe substituiert ist.

2. Eine Verbindung gemäß Anspruch 1, in der R eine $C_{3-10}$-Alkylgruppe, eine Heterocyclylalkylgruppe oder eine $C_{5-8}$-Cycloalkylgruppe darstellt, wobei jede mit einer ketonischen Oxogruppe substituiert ist.

3. Eine Verbindung gemäß Anspruch 1 der Formel (III):

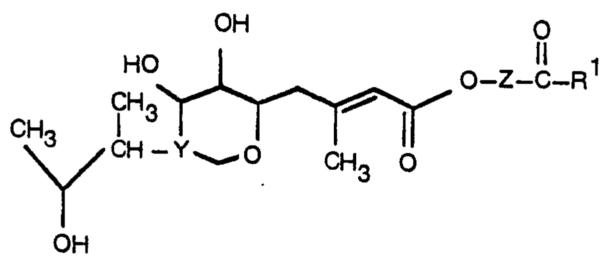

(III)

in der Y in bezug auf Formel (II) definiert ist, Z eine geradkettige oder verzweigte $C_{1-12}$-ALkylengruppe bedeutet und $R^1$ eine $C_{1-10}$-Alkyl-, $C_{3-8}$-Cycloalkyl- $C_{2-10}$-Alkenyl-, Aryl-, Arylalkyl-, Cycloalkylalkyl- oder Heterocyclylgruppe bedeutet.

4. Eine Verbindung gemäß Anspruch 3, in der $R^1$ eine $C_{1-8}$-Alkyl-, Heterocyclyl- oder gegebenenfalls substituierte Phenylgruppe bedeutet und Z eine geradkettige $C_{1-8}$-Alkylengruppe darstellt.

5. Eine Verbindung gemäß einem der Ansprüche 1 bis 4, in der Y eine

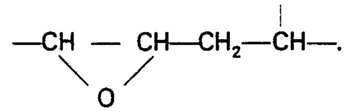

Gruppe bedeutet.

6. Eine pharmazeutische oder veterinäre Zusammensetzung, enthaltend eine Verbindung gemäß einem der Ansprüche 1 bis 5, zusammen mit einem pharmakologisch oder veterinär verträglichen Träger oder Hilfsmittel.

7. Eine Zusammensetzung gemäß Anspruch 6 in Form einer Einzeldosis.

8. Eine Zusammensetzung gemäß Anspruch 6 in Form eines Futtermittels für Tiere oder von Trinkwasservorrat.

9. Ein Verfahren zur Herstellung einer Verbindung der Formel (II), wie in Anspruch 1 definiert, die Maßnahmen umfassend, daß eine Säure der Formel (I):

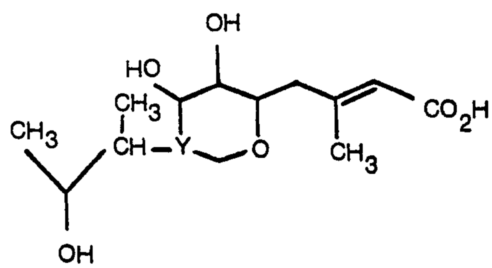

(I)

in der Y 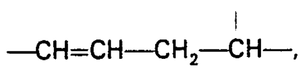, 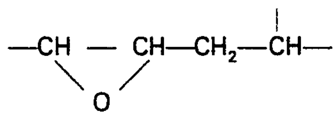 oder

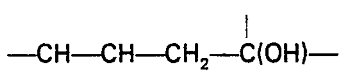

oder ein Salz oder reaktives Derivat davon ist, mit einem Alkohol der Formel ROH, wobei R wie in bezug auf Formel (II) definiert ist, oder einem veresternden Derivat desselben behandelt wird.

10. Ein Verfahren gemäß Anspruch 9, in dem ein Alkalimetallsalz der Säure der Formel (I) mit einem veresterndem Derivat von ROH der Formel R—X umgestzt wird, wobei X ein Halogenatom oder eine Alkyl- oder Arylsulfonatgruppe ist.

11. Ein Verfahren zur Herstellung einer Verbindung der Formel (II) wie in Anspruch 1 definiert, die Maßnahmen umfassend, daß ein symmetrisches oder gemischtes Anhydrid-Derivat einer Säure der Formel (I):

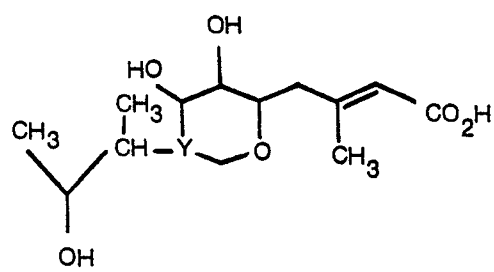

(I)

mit einem Alkohol der Formel ROH oder einem Alkalimetall- oder Erdalkalimetall-alkoxid des Alkohols, in dem R wie in bezug auf Formel (II) definiert ist, behandelt wird.

12. Ein Verfahren zur Herstellung einer Verbindung der Formel (II), wie in Anspruch 1 definiert, die Maßnahmen umfassend, daß eine Verbindung der Formel (IV):

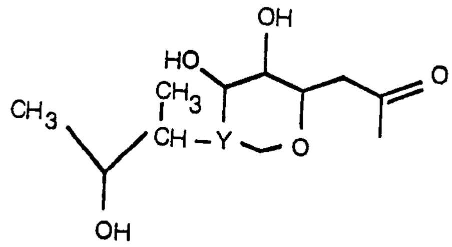

(IV)

in der Y wie in bezug auf Formel (II) definiert ist, mit einer Verbindung der Formel (V) oder (VI) umgesetzt wird:

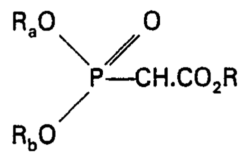

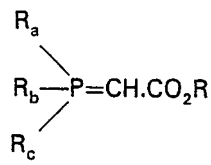

(V) (VI)

in denen $R_a$, $R_b$ und $R_c$ gleich oder verschieden sind und je eine niedere Alkyl-, Aryl- oder Arylalkylgruppe sind, und R wie in bezug auf Formel (II) definiert ist.

13. Ein Verfahren gemäß einem der Ansprüche 9 bis 12, in dem die Hydroxylgruppen in den Verbindungen der Formeln (I) und (IV) am Anfang geschützt sind und anschließend entfernt werden.